(19) Europäisches Patentamt
European Patent Office
Office européen des brevets

(11) Publication number: **0 464 311 A2**

# EUROPEAN PATENT APPLICATION

(21) Application number: **91104355.2**

(22) Date of filing: **20.03.91**

(51) Int. Cl.5: **A61K 35/84**

(30) Priority: **04.07.90 JP 176559/90**

(43) Date of publication of application:
**08.01.92 Bulletin 92/02**

(84) Designated Contracting States:
**AT BE CH DE DK ES FR GB GR IT LI LU NL SE**

(71) Applicant: **NODA SHOKUKIN KOGYO CO., LTD.**
**121, Shimizu**
**Noda-shi Chiba-ken(JP)**

(72) Inventor: **Iizuka, Chiyokichi**
**905-10, Shimizu**
**Noda-shi, Chiba-ken(JP)**
Inventor: **Ohashi, Yasuhiro**
**1-51-11, Iwana**
**Noda-shi, Chiba-ken(JP)**
Inventor: **Suzuki, Fumiko**
**436-5, Komejima,Showa-machi**
**Kitakatsushika-gun, Saitama-ken(JP)**

(74) Representative: **Wey, Hans-Heinrich, Dipl.-Ing.**
**et al**
**Patentanwälte Wey & Partner**
**Widenmayerstrasse 49**
**W-8000 München 22(DE)**

(54) **Anti-virus substance and process for producing the same.**

(57) An anti-virus substance essentially consisting of water-soluble modified lignin containing sugars and proteins extracted from basidiomycetes such as Lentinus edodes cultured in a medium essentially consisting of a material derived from a plant containing lignin. This substance is effective in preventing viral infection in both plants and animals.

EP 0 464 311 A2

## TECHNICAL FIELD

The present invention relates to an anti-virus substance derived by culturing hypha of basidiomycetes, and a process for producing the same.

## BACKGROUND OF THE INVENTION

In medical and agricultural fields, various forms of virus infection are becoming an increasing concern in recent years.

For instance, B-type hepatitis and C-type hepatitis are caused by B-type and C-type hepatitis viruses, respectively, which are responsible for the liver failure of the patient, and this liver failure leads to chronic hepatitis, liver cirrhosis and even liver cancer, sometimes eventually resulting in the death of the patient.

Human AIDS virus has an affinity with human $T_4^+$ T cells, and can kill them by infection. As a result, the patient develops a deficiency in his immunity system which in turn causes an acquired immunity deficiency syndrome, and almost 100% of the patients who showed such symptoms resulted in death in a few years to 10 years.

Also, human herpes viruses (type I and type II) are known to cause various ailments such as stomatitis, genital herpes, keratitis, meningitis, and the infection of upper and lower airway.

Currently, almost no effective curing agents are available against such viral diseases, and such known curing agents as interferon, adeninearabinoside, azidothymidine and aciclovir are known to have various problems such as excessively strong side effects. Totally no substance is available for use on plants with any effectiveness and with such a low toxic level as to allow it to be sprayed in large quantities.

Therefore, in preventing such viral diseases, it is a prerogative to prevent infection by virus, but, since predicting infection by virus is extremely difficult, it is strongly desired to obtain an agent for preventing viral infection with sufficient safety and effectiveness in regard to a wide spectrum of viruses.

The inventors of this application have conducted a research on substances extracted from the cultured hypha of basidiomycetes such as Lentinus edodes (shiitake), and have so far discovered that substances extracted from cultured hypha have the function of activating immunity (Japanese patent publication No. 53-23392), that substances extracted from cultured hypha having molecular weights of six million to 1.5 million (A fraction) and 1.5 million to eight hundred thousand (B fraction) are effective against B-type hepatitis (Japanese patent laid open publication No. 62-70532), and that a composite cytokinin activating substance essentially consisting of polysaccharide and a zeatin related material extracted from cultured hypha is effective as an anti-virus agent (Japanese patent publication No. 62-36009).

## BRIEF SUMMARY OF THE INVENTION

The substance extracted from the cultured hypha of basidiomycetes such as Lentinus edodes demonstrates a high level of anti-virus activity, and is totally free from side effects because it is not toxic.

However, the process of isolating the effective component of the substance extracted from the cultured hypha is extremely complex, and its yield ratio is so low that the isolation process does not contribute to the enhancement of its effect.

A primary object of the present invention is to provide an anti-virus substance which is more effective than conventional substances, by efficiently extracting a component demonstrating a high level of anti-virus effect from the cultured hypha of basidiomycetes such as Lentinus edodes, and a process for producing the same.

To achieve such an object, the inventors of this application completed this invention by simply and effectively extracting a component having an anti-virus effectiveness from the cultured hypha of basidiomycetes such as Lentinus edodes by such means as hot water extraction and ultra-filtration, closely analyzing this substance, and finding that this substance essentially consists of water soluble lignin to which sugars and proteins are attached.

In other words, the anti-virus substance of the present invention essentially consists of water-soluble modified lignin containing sugars and proteins extracted from basidiomycetes cultured in a medium essentially consisting of a material derived from a plant containing lignin.

The process of producing an anti-virus substance of the present invention comprises the steps of culturing basidiomycetes by using a culture medium consisting of a material derived from a plant containing lignin, and extracting a component which is rich in water-soluble modified lignin containing sugar and protein from the cultured hypha of basidiomycetes.

## DETAILED DESCRIPTION OF THE PREFERRED EMBODIMENTS

Now the present invention is described in the following in terms of its preferred embodiments.

The basidiomycetes of the present invention may consist of various edible and medically effective fungi such as Lentinus edodes (shiitake), Coriolus versicolor (kawaratake), Pleurotus ostreatus (hiratake), Flammulina velutipes (enokitake), Ganoderma lucidum (manentake), Grifola frondosa (maitake) and other edible and medically useful fungi, but most preferably consists of shiitake.

According to the present invention, the hypha of such basidiomycetes is cultured, and an effective component is extracted therefrom. In this case, the culture medium for the hypha may be either solid or liquid, but is required to contain a material derived from a plant containing lignin. The plant containing lignin is preferred to be a plant belonging to gramineae or true grasses, and the material may consist of bagasse, barley straws, rice straws, corn stalks and leaves, rice bran, wheat bran, and so forth. A particularly preferred culture medium consists mainly of bagasse and optionally contains such nutrients as rice bran, saw dust, pepton, yeast, potatoes, molasses and so forth.

The culturing of the hypha of basidiomycetes can be dome by inoculating, in such a culture medium as mentioned above, pellets of hypha obtained by culturing the spores of basidiomycetes in a liquid culture medium. In the case of a solid culture, after inoculating the hypha, it is placed in a culture chamber kept at the temperature of 10 to 25 °C and the humidity of 50 to 90% for three to six months. Ideally, it should be placed in a culture chamber kept at the temperature of 20 to 25 °C and the humidity of 60% for four to six months. Preferably, the culture medium in which the hypha has multiplied is moved to a temperature processing room in which it is subjected to a controlled temperature condition which may consist of heating it to 32 to 34 °C for 24 to 48 hours, and is cooled in a low temperature chamber at 4 to 8 °C and 85% condition for five to seven days. This temperature processing is utilized for the purpose of improving the stability of the quality of the product, and is not essential to the present invention. Thereafter, as the cultured medium is placed in a culture chamber, the generation of fruiting bodies begins. The culturing is terminated at this point, and the culture is pulverized by a crusher as described hereinafter. In the case of a liquid culturing medium, the culturing is based on either the aerobic culturing or the shake culturing which lasts for one week to one month at the temperature condition of 15 to 30 °C. The culturing is terminated when the culture medium is substantially filled by the hypha.

Upon completion of the culturing, the hypha is subjected to autolysis by utilizing the enzyme existing in the hypha, and a cultured ingredient is extracted therefrom. According to a preferred process, the cultured material is pulverized, and is subjected to autolysis by the enzyme existing in the hypha by maintaining the pulverized material at 40 to 90 °C for three to six hours. Then, hot water of 40 °C or higher is poured over the pulverized material to extract an effective ingredient therefrom. The suspension liquid thus obtained is filled into a flannel filter bag, and is pressurized for filtration. The filtered liquid is further filtered with a membrane filter to remove microbes and obtain an extraction fluid containing an effective ingredient. In the case of a liquid culture medium, after the hypha is pulverized if necessary, it is heated to 40 to 60 °C for autolysis to obtain suspended liquid cultured material in which hypha is dissolved. This cultured material is filtered and removed of microbes as described above to obtain an extraction liquid.

In producing the anti-virus substance of the present invention, it is preferred to further purify the thus obtained extraction liquid into a component rich in water soluble lignin. This purification process may consist of an ultra filtration separation process. When ultra filtration is carried out by using filter membranes for the molecular weights of 10,000 and 200,000, respectively, a high activity level against animal herpes virus was found in the fraction having the molecular weight in excess of 200,000. The yield ratio from the extraction liquid was 10% on average.

It was found that the thus obtained anti-virus substance is a polysuccaride-protein complex material containing lignin.

According to this production process, inorganic substances are contained in the effective ingredient of the extraction liquid in a relatively high level and, since such substances generally belong to the fraction having the molecular weight of 10,000 or lower, they can be separated very easily.

According to these results, it was found that the effective anti-virus component consisted of the following ingredients:

| sample | sugar | protein | lignin | inorganic | total |
|--------|-------|---------|--------|-----------|-------|
| original | 40.8 | 11.7 | 35.7 | 14.5 | 102.7 |
| over 200,000 | 66.2 | 6.4 | 47.7 | 3.0 | 123.3 |
| 10,000 – 200,000 | 71.0 | 7.8 | 42.2 | 4.6 | 125.6 |
| below 10,000 | 35.0 | 11.1 | 24.7 | 15.3 | 86.1 |

sugar: phenol-sulfuric acid method (glucose stand. 480 nm)
protein: semimicro Kjerdahl method
lignin: acetylbromide method (absorption constant 20)
inorganic: direct ashing method

It is believed that the substance of the present invention extracted from cultured hypha of Lentinus edodes or containing such a fraction hampers the coupling between various viruses and their target cells, and thereby prevent viral infection as can be seen from the embodiments described hereinafter.

According to the method of the present invention for producing the anti-virus substance, the component effective for the anti-virus action can be effectively extracted and separated, and the substance which is thus obtained from natural materials is free from side effects as opposed to those produced from synthetic chemical compositions.

Embodiment 1

(1) Culturing of the hypha of Lentinus edodes

A mixture of 90% bagasse, 5% rice bran and 5% other nourishing ingredients such as wheat bran placed in a solid culture medium was sterilized, and Lentinus edodes was inoculated therein. Thereafter, the culture medium was moved to a culture chamber which was kept at the temperature of 20 to 25 °C and the humidity of 60%, and was placed therein for three to six months. After the hypha had multiplied in the culture medium, it was moved to a temperature processing chamber in which the culture medium was heated to 32 to 34 °C for 24 to 48 hours. Then, the culture medium was moved to a low temperature processing chamber which was kept at the temperature of 5 to 8 °C and the humidity of 85%, and was kept therein for five to seven days. Further, the culture medium was placed in a growing chamber, and, once the fruiting bodies had started be produced on the surface of the culture medium, the culture medium was taken out to be pulverized by a pulverizer.

(2) Extraction of an effective component from the cultured material

The pulverized material was heated in the presence of air for three to four hours at the temperature of approximately 80 °C to promote the autolysis of the hypha on the one hand and to dry it to the water content of 3 to 5% on the other hand. Approximately 5 liters of water was added to 600 g of the pulverized material, and the mixture was boiled for one hour while it was being stirred. As a result of this constant stirring, the substance produced by the metabolism of the hypha as well as the effective component contained in the intercellular fluid of the hypha dissolved into the water. The thus obtained suspension fluid was filled into a flannel bag, and was filtered under pressure. The filtered liquid was further filtered by a membrane filter to remove microbes therefrom. The liquid thus extracted was concentrated by freeze-drying, and was converted into brown powder.

According to our analysis, the contents of the powder consisted of 34.0% sugar, 10.8% protein, 43.0% water-soluble lignin, and 12.2% others. The sugar content was evaluated by the phenol-sulfuric acid method, the protein content was evaluated by the semimicro Kjerdahl method, and the lignin content was evaluated by the acetylbromide method.

4

## (3) Fractionation of the effective component

Ultra filtration was conducted on the extraction liquid itself or after it was concentrated using a ultra filtration membrane for the molecular weight of 200,000 (which is referred to as Fr. 1 hereinafter).

Then, the filtered liquid was further filtered by using an ultra filtration membrane for the molecular weight of 10,000, and we obtained a fraction whose molecular weight ranged between 200,000 and 10,000 (Fr. 2) and a fraction whose molecular weight was less than 10,000 (Fr. 3). Fr. 1 through Fr. 3 were concentrated and freeze-dried to obtain brown powder. The contents were as mentioned earlier.

## Embodiment 2

The effectiveness of the extraction liquid and the fractions in preventing the absorption of virus was evaluated.

MDBK cells derived from a cow kidney was grown in a 24-hole plate for tissue culturing. It was grown in a culture medium consisting of Eagle MEM containing 2% plasma so that 50 to 100 plaque formation units may be contained in 0.2 milliliter. The extraction liquid itself, a fraction thereof diluted in various concentrations, and drugs to be compared in various concentrations were mixed with an equal amount of diluted virus solution and were allowed to undergo a chemical reaction at 4 °C for one hour. After washing the cells in the plate with phosphate buffer saline (PBS) prescribed by Double Bekko, 0.2 milliliter of the reaction fluid was placed in each hole, and was allowed to contact the cells for one hour. After washing the cells with PBS, the culture containing 0.2% of agar was layered and fixed, the culture medium was placed in a carbon dioxide incubator for three days. The plaque count was carried out by fixing the cells in each hole with 1 milliliter of 10% formalin, and dying them 0.5 milliliter of 0.03% methylene blue. The plaque prevention ratios of the extracted liquid and the different fractions at various concentrations were computed, and the obtained results of $ED_{50}(\mu g/ml)$ are given in the following.

| | $ED_{50}(\mu g/ml)$ |
|---|---|
| extracted liquid (original) | 55.1 |
| Fr. 1 (> 200,000) | 13.0 |
| Fr. 2 (10,000 to 200,000) | 110.0 |
| Fr. 3 (< 10,000) | invalid |

These results indicate that the ultra filtrated fraction having the molecular weight of 200,000 or higher is most effective in preventing IBRV infection.

The substance of the present invention demonstrates a high anti-virus activity, and is highly safe without any toxicity or side effects.

Further, according to the method of the present invention, since the substance which can prevent viral infection can be separated and recovered in a simple and efficient manner, it would be widely applicable in preventing animal and plant viral infections.

Although the present invention has been described in terms of specific embodiments, it is obvious to a person skilled in the art that various modifications and alterations are possible without departing from the spirit of the present invention which is set forth in the appended claims.

## Claims

1.  An anti-virus substance essentially consisting of water-soluble modified lignin containing sugar and protein extracted from basidiomycetes cultured in a medium essentially consisting of a material derived from a plant containing lignin.

2.  An anti-virus substance according to claim 1 which is derived as hypha of basidiomycetes.

3.  An anti-virus substance according to claim 1, including a substance extracted from autolytically cultured hypha of basidiomycetes by using hot water, or a substance containing a purified fraction obtained therefrom.

4. A process of producing an anti-virus substance essentially comprising the steps of culturing basidiomycetes by using a culture medium consisting of a material derived from a plant containing lignin, and extracting a component which is rich in water-soluble modified lignin containing sugar and protein from the cultured hypha of basidiomycetes.

5. A process of producing an anti-virus substance according to claim 4, consisting of the use of the hypha of basidiomycetes.

6. A process of producing an anti-virus substance according to claim 4, consisting of the use of a substance extracted from autolytically cultured hypha of basidiomycetes by using hot water, or a substance containing a fraction purified therefrom.